# FASCICULE DE BREVET EUROPEEN

(11) **EP 0 981 322 B1**
(45) Date de publication et mention de la délivrance du brevet: **10.09.2003**
(21) Numéro de dépôt: 98949073.5
(22) Date de dépôt: 16.10.1998
(51) Int. Cl.: A61K 7/13

(54) **COMPOSITION DE TEINTURE D'OXYDATION DES FIBRES KERATINIQUES**
ZUSAMMENSETZUNG FÜR DIE OXIDATIONSFÄRBUNG VON KERATINISCHEN FASERN
OXIDATION DYEING FOR KERATIN FIBRES

(30) Priorité: 22.10.1997 FR 9713243
(43) Date de publication de la demande: 01.03.2000
(73) Titulaire: L'OREAL, 75008 Paris (FR)
(72) Inventeur: LANG, Gérard, F-95390 Saint Prix (FR); COTTERET, Jean, F-78480 Verneuil sur Seine (FR)
(74) Mandataire: Goulard, Sophie
(86) Numéro de dépôt international: FR9802231
(87) Numéro de publication internationale: WO99020236

(56) Documents cités:
- EP-A- 0 310 675
- EP-A- 0 716 846
- EP-A- 0 795 313
- WO-A-97/37633

## Description

L'invention a pour objet une composition pour la teinture d'oxydation des fibres kératiniques, et en particulier des fibres kératiniques humaines telles que les cheveux comprenant, dans un milieu approprié pour la teinture, au moins un colorant auto-oxydable, et au moins une enzyme de type oxydo-réductase à 2 électrons en présence d'au moins un donneur pour ladite enzyme, ainsi que le procédé de teinture mettant en oeuvre cette composition.

Il est connu de teindre les fibres kératiniques et en particulier les cheveux humains avec des compositions tinctoriales contenant des colorants auto-oxydables tels que des dérivés benzéniques comportant au moins trois groupements hydroxyle et/ou amino et des dérivés indoliques, tels que le 5,6-dihydroxy indole. Ces colorants auto-oxydables ont la particularité de pouvoir s'oxyder sans autre agent oxydant que l'oxygène de l'air, pour donner naissance à des molécules colorées et colorantes. Cependant les colorations obtenues en mettant en oeuvre ces colorants ne sont pas toujours satisfaisantes notamment du point de vue de leur puissance et de leur chromaticité.

L'oxydation de ces colorants auto-oxydables peut être favorisée en utilisant, généralement en milieu alcalin, un agent oxydant classique tel que par exemple le peroxyde d'hydrogène. L'utilisation des milieux alcalins en présence de peroxyde d'hydrogène présente pour inconvénient d'entraîner une dégradation non négligeable des fibres, ainsi qu'une décoloration des fibres kératiniques qui n'est pas toujours souhaitable.

Il a déjà été proposé de teindre les fibres kératiniques, notamment dans la demande de brevet EP-A-0 310 675, avec des compositions comprenant un précurseur de colorant d'oxydation de type benzénique, en association avec des enzymes telles que la pyranose-oxydase, la glucose-oxydase ou bien l'uricase, en présence d'un donneur pour lesdites enzymes. Ces procédés de teinture, bien qu'étant mis en oeuvre dans des conditions n'entraînant pas une dégradation des fibres kératiniques comparable à celle engendrée par les teintures réalisées en présence de peroxyde d'hydrogène, conduisent à des colorations ne donnant pas entière satisfaction notamment du point de vue de la puissance des colorations obtenues.

Or, la demanderesse vient maintenant de découvrir qu'il est possible d'obtenir de nouvelles teintures qui, tout en étant exemptes de base d'oxydation, sont capables de conduire à des colorations puissantes sans engendrer de dégradation significative, ni de décoloration importante des fibres kératiniques, peu sélectives et résistant bien aux diverses agressions que peuvent subir les cheveux, en associant au moins un colorant auto-oxydable, et au moins une enzyme de type oxydo-réductase à 2 électrons en présence d'au moins un donneur pour ladite enzyme.

Cette découverte est à la base de la présente invention.

L'invention a donc pour premier objet une composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :
- au moins un colorant auto-oxydable,
- au moins une enzyme de type oxydo-réductase à 2 électrons,
- et au moins un donneur pour ladite enzyme ;
ladite composition étant exempte de base d'oxydation.

La composition tinctoriale prête à l'emploi conforme à l'invention conduit à des colorations puissantes, chromatiques, présentant une faible sélectivité et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture d'oxydation des fibres kératiniques mettant en oeuvre cette composition tinctoriale prête à l'emploi.

La ou les oxydo-réductases à 2 électrons utilisées dans la composition tinctoriale prête à l'emploi conforme à l'invention peuvent notamment être choisies parmi les pyranose oxydases, les glucose oxydases, les glycérol oxydases, les lactates oxydases, les pyruvate oxydases, et les uricases.

Selon l'invention, l'oxydo-réductase à 2 électrons est de préférence choisie parmi les uricases d'origine animale, microbiologique ou biotechnologique.

A titre d'exemple, on peut notamment citer l'uricase extraite de foie de sanglier, l'uricase d'Arthrobacter globiformis, ainsi que l'uricase d'Aspergillus flavus.

La ou les oxydo-réductases à 2 électrons peuvent être utilisées sous forme cristalline pure ou sous une forme diluée dans un diluant inerte pour ladite oxydo-réductase à 2 électrons.

La ou les oxydo-réductases à 2 électrons conformes à l'invention représentent de préférence de 0,01 à 20 % en poids environ du poids total de la composition tinctoriale prête à l'emploi, et encore plus préférentiellement de 0,1 à 5 % en poids environ de ce poids.

Selon l'invention, on entend par donneur, les différents substrats participant au fonctionnement de ladite ou desdites oxydo-réductases à 2 électrons.

La nature du donneur (ou substrat) pour ladite enzyme varie en fonction de la nature de l'oxydo-réductase à 2 électrons qui est utilisée. Par exemple, à titre de donneur pour les pyranose oxydases, on peut citer le D-glucose, le L-sorbose et le D-xylose ; à titre de donneur pour les glucose oxydases, on peut citer le D-glucose, à titre de donneur pour les glycérol oxydases, on peut citer le glycérol et la dihydroxyacétone ; à titre de donneur pour les lactate oxydases, on peut citer l'acide lactique et ses sels ; à titre de donneur pour les pyruvate oxydases, on peut citer l'acide pyruvique et ses sels ; et enfin à titre de donneur pour les uricases, on peut citer l'acide urique et ses sels.

Le ou les donneurs (ou substrats) utilisés conformément à l'invention représentent de préférence de 0,01 à 20 % en poids environ du poids total de la composition tinctoriale prête à l'emploi conforme à l'invention et encore plus préférentiellement de 0,1 à 5 % en environ de ce poids.

La nature du ou des colorants auto-oxydables utilisés dans la composition tinctoriale prête à l'emploi n'est pas critique. Ils peuvent notamment être choisis parmi les colorants auto-oxydables benzéniques, indoliques ou indoliniques.

Parmi les colorants auto-oxydables benzéniques utilisables dans la composition tinctoriale conforme à l'invention, on peut notamment citer les composés de formule (I) suivante et leurs sels d'addition avec un acide : 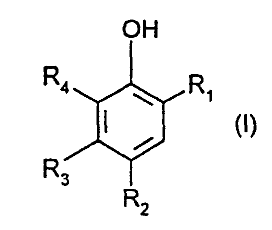 dans laquelle :
- R₁ représente un atome d'hydrogène, un radical alkyle en C₁-C₄ ou un radical amino,
- R₂ représente un radical alkyle en C₁-C₄, hydroxyle, amino, monoalkyl(C₁-C₄)amino ou dialkyl(C₁-C₄)amino,
- R₃ représente un atome d'hydrogène, un radical hydroxyle ou amino,
- R₄ représente un atome d'hydrogène ou un radical amino ;
étant entendu qu'au moins deux des radicaux R₁ à R₄ représentent, indépendamment l'un de l'autre, un radical hydroxyle, amino, monoalkyl(C₁-C₄)amino ou dialkyl(C₁-C₄)amino.

Parmi les colorants auto-oxydables benzéniques de formule (I) ci-dessus, on peut plus particulièrement citer le 1,2,4-trihydroxybenzène, le 1-méthyl 2,4,5-trihydroxybenzène, le 2,4-diamino 6-méthyl phénol, le 2-amino 4-méthylamino phénol, le 2,5-diamino 4-méthyl phénol, le 2,6-diamino 4-diéthylamino phénol, le 2,6-diamino 1,4-dihydroxy benzène, et leurs sels d'addition avec un acide.

Parmi les colorants auto-oxydables indoliques et indoliniques utilisables dans la composition tinctoriale conforme à l'invention, on peut notamment citer les composés de formules (II) et (III) suivantes, et leurs sels d'addition avec un acide : 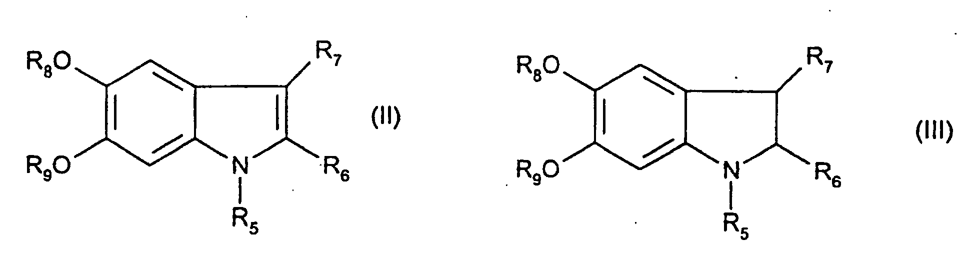 dans lesquelles :
- R₅, R₇, R₈ et R₉, identiques ou différents, représentent un atome d'hydrogène, un radical alkyle en C₁-C₄ ou acyle en C₁-C₄,
- R₆ représente un atome d'hydrogène, un radical alkyle en C₁-C₄ ou un radical carboxyle.

Parmi les colorants auto-oxydables de formule (II) ci-dessus, on peut plus particulièrement citer le 5,6-dihydroxy indole, le 2-méthyl 5,6-dihydroxy indole, le 3-méthyl 5,6-dihydroxy indole, le 1-méthyl 5,6-dihydroxy indole, le 2,3-diméthyl 5,6-dihydroxy indole, le 5-méthoxy 6-hydroxyindole, le 5-acétoxy 6-hydroxy indole, le 5,6-diacétoxy indole, l'acide 5,6-dihydroxy indole 2-carboxylique, et leurs sels d'addition avec un acide.

Parmi les colorants auto-oxydables de formule (III) ci-dessus, on peut plus particulièrement citer la 5,6-dihydroxy indoline, la 1-méthyl 5,6-dihydroxy indoline, la 1-éthyl 5,6-dihydroxy indoline, et leurs sels d'addition avec un acide.

Le ou les colorants auto-oxydables représentent de préférence de 0,0005 à 12 % en poids environ du poids total de la composition tinctoriale conforme à l'invention, et encore plus préférentiellement de 0,005 à 8 % en poids environ de ce poids.

D'une manière générale, les sels d'addition avec un acide utilisables dans le cadre des compositions tinctoriales de l'invention (colorants auto-oxydables) sont notamment choisis parmi les chlorhydrates, les bromhydrates, les sulfates et les tartrates, les lactates et les acétates.

La composition tinctoriale prête à l'emploi conforme à l'invention peut en outre renfermer un ou plusieurs colorants directs notamment pour modifier les nuances ou les enrichir en reflets.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Le pH de la composition prête à l'emploi conforme à l'invention est choisi de telle manière que l'activité enzymatique de l'oxydo-réductase à 2 électrons soit suffisante. Il est généralement compris entre 5 et 11 environ, et de préférence entre 6,5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (IV) suivante : 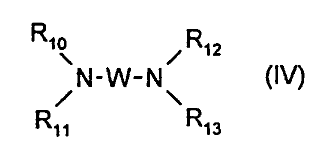 dans laquelle W est un reste propylène éventuellement substitué par un groupement hydroxyle ou un radical alkyle en C₁-C₄ ; R₁₀, R₁₁, R₁₂ et R₁₃, identiques ou différents, représentent un atome d'hydrogène, un radical alkyle en C₁-C₄ ou hydroxyalkyle en C₁-C₄.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des enzymes différentes des oxydo-réductases à 2 électrons utilisées conformément à l'invention telles que par exemples des peroxydases, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, le ou les colorants auto-oxydables et la ou les oxydo-réductases à 2 électrons sont présents au sein de la même composition prête à l'emploi, et par conséquent ladite composition doit être exempte d'oxygène gazeux, de manière à éviter toute oxydation prématurée du ou des colorants auto-oxydables.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, le procédé comporte une étape préliminaire consistant à stocker sous forme séparée, d'une part, une composition(A) comprenant, dans un milieu approprié pour la teinture, au moins un colorant auto-oxydable et, d'autre part, une composition (B) renfermant, dans un milieu approprié pour la teinture, au moins une enzyme de type oxydo-réductase à 2 électrons en présence d'au moins un donneur pour ladite enzyme, puis à procéder à leur mélange au moment de l'emploi avant d'appliquer ce mélange sur les fibres kératiniques.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES 1 à 3 DE TEINTURE

On a préparé les compositions tinctoriales prêtes à l'emploi suivantes (teneurs en grammes) :

| **COMPOSITION** | **1** | **2** | **3** |
|---|---|---|---|
| 5,6-dihydroxy indole (colorant auto-oxydable) | 0,8 | - | - |
| Monobromhydrate de 5,6-dihydroxy indoline (colorant auto-oxydable) | - | 1,2 | - |
| 1,2,4-trihydroxy benzène (colorant auto-oxydable) | - | - | 1,0 |
| Uricase d'Arthrobacter globiformis à 20 Unités Internationales (U.l.) / mg, commercialisée par la société Sigma | 1,5 | 1,5 | 1,5 |
| Acide urique | 1,5 | 1,5 | 1,5 |
| Support de teinture commun (*) | (*) | (*) | (*) |
| Eau déminéralisée q.s.p. | 100 g | 100 g | 100 g |

| | | | |
|---|---|---|---|
| (*) : Support de teinture commun : - Ethanol 20,0 g - Hydroxyéthylcellulose vendue sous la dénomination NATROSOL 250 HR ® par la société AQUALON 1,0 g - Alkyl (C₈-C₁₀) polyglucoside en solution aqueuse à 60 % de matière active (M.A.) tamponné par du citrate d'ammonium (0,5%), vendu sous la dénomination ORAMIX CG110 ® par la société SEPPIC 8,0 g - Monoéthanolamine q.s. pH = 9,5 | | | |

Chacune des compositions tinctoriales prêtes à l'emploi décrites ci-dessus a été appliquée sur des mèches de cheveux gris naturels à 90 % de blancs pendant 30 minutes. Les cheveux ont ensuite été rincés, lavés avec un shampooing standard, puis séchés.

Les cheveux ont été teints dans les nuances figurant dans le tableau ci-après :

| **EXEMPLE** | **Nuance obtenue** |
|---|---|
| **1** | Blond cendré |
| **2** | Blond |
| **3** | Blond nacré |

## Revendications

1. Composition prête à l'emploi, pour la teinture d'oxydation des fibres kératiniques et en particulier des fibres kératiniques humaines telles que les cheveux, **caractérisée par le fait qu'**elle comprend, dans un milieu approprié pour la teinture :
- au moins un colorant auto-oxydable,
- au moins une enzyme de type oxydo-réductase à 2 électrons,
- et au moins un donneur pour ladite enzyme ;
ladite composition étant exempte de base d'oxydation.

2. Composition selon la revendication 1, **caractérisée par le fait que** l'oxydo-réductase à 2 électrons est choisie parmi les pyranose oxydases, les glucose oxydases, les glycérol oxydases, les lactates oxydases, les pyruvate oxydases, et les uricases.

3. Composition selon l'une quelconque des revendications 1 ou 2, **caractérisée par le fait que** l'oxydo-réductase à 2 électrons est choisie parmi les uricases d'origine animale, microbiologique ou biotechnologique.

4. Composition selon l'une quelconque des revendications précédentes, **caractérisée par le fait que** la ou les oxydo-réductases à 2 électrons représentent de 0,01 à 20 % en poids du poids total de la composition tinctoriale prête à l'emploi.

5. Composition selon la revendication 4, **caractérisée par le fait que** la ou les oxydo-réductases à 2 électrons représentent de 0,1 à 5 % en poids du poids total de la composition tinctoriale prête à l'emploi.

6. Composition selon l'une quelconque des revendications 3 à 5, **caractérisée par le fait que** le donneur (ou substrat) pour ladite oxydo-réductase à 2 électrons est choisi parmi l'acide urique et ses sels.

7. Composition selon l'une quelconque des revendications précédentes, **caractérisée par le fait que** le ou les donneurs représentent de 0,01 à 20 % en poids du poids total de la composition tinctoriale prête à l'emploi.

8. Composition selon la revendication 7, **caractérisée par le fait que** le ou les donneurs représentent de 0,1 à 5 % en poids du poids total de la composition tinctoriale prête à l'emploi.

9. Composition selon l'une quelconque des revendications précédentes, **caractérisée par le fait que** le ou les colorants auto-oxydables sont choisis parmi les colorants auto-oxydables benzéniques, indoliques ou indoliniques.

10. Composition selon la revendication 9, **caractérisée par le fait que** les colorants auto-oxydables benzéniques sont choisis parmi les composés de formule (I) suivante et leurs sels d'addition avec un acide : 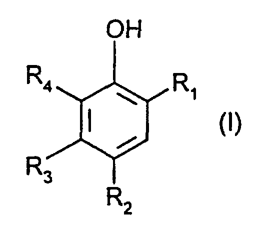 dans laquelle :
- R₁ représente un atome d'hydrogène, un radical alkyle en C₁-C₄ ou un radical amino,
- R₂ représente un radical alkyle en C₁-C₄, hydroxyle, amino, monoalkyl(C₁-C₄)amino ou dialkyl(C₁-C₄)amino,
- R₃ représente un atome d'hydrogène, un radical hydroxyle ou amino,
- R₄ représente un atome d'hydrogène ou un radical amino ;
étant entendu qu'au moins deux des radicaux R₁ à R₄ représentent, indépendamment l'un de l'autre, un radical hydroxyle, amino, monoalkyl(C₁-C₄)amino ou dialkyl(C₁-C₄)amino.

11. Composition selon la revendication 10, **caractérisée par le fait que** les colorants auto-oxydables benzéniques de formule (I) sont choisis parmi le 1,2,4-trihydroxybenzène, le 1-méthyl 2,4,5-trihydroxybenzène, le 2,4-diamino 6-méthyl phénol, le 2-amino 4-méthylamino phénol, le 2,5-diamino 4-méthyl phénol, le 2,6-diamino 4-diéthylamino phénol, le 2,6-diamino 1,4-dihydroxy benzène, et leurs sels d'addition avec un acide.

12. Composition selon la revendication 9, **caractérisée par le fait que** les colorants auto-oxydables indoliques et indoliniques sont choisis parmi les composés de formules (II) et (III) suivantes, et leurs sels d'addition avec un acide: 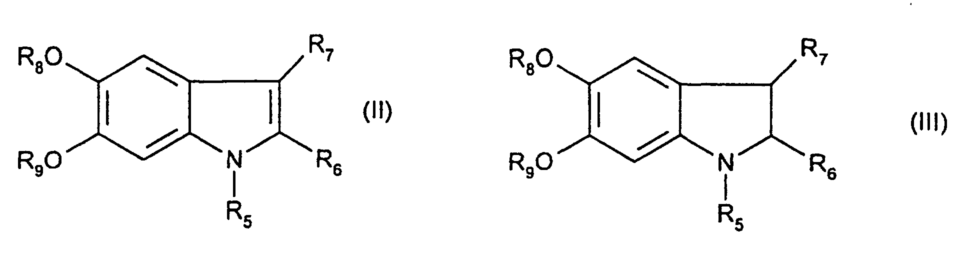 dans lesquelles :
- R₅, R₇, R₈ et R₉, identiques ou différents, représentent un atome d'hydrogène, un radical alkyle en C₁-C₄ ou acyle en C₁-C₄,
- R₆ représente un atome d'hydrogène, un radical alkyle en C₁-C₄ ou un radical carboxyle.

13. Composition selon la revendication 12, **caractérisée par le fait que** les colorants auto-oxydables de formule (II) sont choisis parmi le 5,6-dihydroxy indole, le 2-méthyl 5,6-dihydroxy indole, le 3-méthyl 5,6-dihydroxy indole, le 1-méthyl 5,6-dihydroxy indole, le 2,3-diméthyl 5,6-dihydroxy indole, le 5-méthoxy 6-hydroxyindole, le 5-acétoxy 6-hydroxy indole, le 5,6-diacétoxy indole, l'acide 5,6-dihydroxy indole 2-carboxylique, et leurs sels d'addition avec un acide.

14. Composition selon la revendication 12, **caractérisée par le fait que** les colorants auto-oxydables de formule (III) sont choisis parmi la 5,6-dihydroxy indoline, la 1-méthyl 5,6-dihydroxy indoline, la 1-éthyl 5,6-dihydroxy indoline, et leurs sels d'addition avec un acide.

15. Composition selon l'une quelconque des revendications précédentes, **caractérisée par le fait que** le ou les colorants auto-oxydables représentent de 0,0005 à 12 % en poids du poids total de la composition tinctoriale.

16. Composition selon la revendication 15, **caractérisée par le fait que** le ou les colorants auto-oxydables représentent de 0,005 à 8 % en poids du poids total de la composition tinctoriale.

17. Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

18. Composition selon l'une quelconque des revendications précédentes, **caractérisée par le fait que** le milieu approprié pour la teinture est constitué par de l'eau ou par un mélange d'eau et d'au moins un solvant organique.

19. Composition selon l'une quelconque des revendications précédentes, **caractérisée par le fait qu'**elle présente un pH compris 5 et 11.

20. Composition selon l'une quelconque des revendications précédentes, **caractérisée par le fait qu'**elle contient au moins une peroxydase.

21. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications précédentes, pendant un temps suffisant pour développer la coloration désirée.

22. Procédé selon la revendication 21, **caractérisé par le fait qu'**il comporte une étape préliminaire consistant à stocker sous forme séparée, d'une part, une composition (A) comprenant, dans un milieu approprié pour la teinture, au moins un colorant auto-oxydable et, d'autre part, une composition (B) renfermant, dans un milieu approprié pour la teinture, au moins une enzyme de type oxydo-réductase à 2 électrons en présence d'au moins un donneur pour ladite enzyme, puis à procéder à leur mélange au moment de l'emploi avant d'appliquer ce mélange sur les fibres kératiniques.

23. Dispositif à plusieurs compartiments ou "kit" de teinture, **caractérisé par le fait qu'**il comporte un premier compartiment renfermant la composition (A) telle que définie dans la revendication 22 et un second compartiment renfermant la composition (B) telle que définie dans la revendication 22.

## Claims

1. Ready-to-use composition for the oxidation dyeing of keratin fibres, and in particular human keratin fibres such as the hair, **characterized in that** it comprises, in a medium which is suitable for dyeing:
- at least one self-oxidizing dye,
- at least one enzyme of 2-electron oxidoreductase type, and
- at least one donor for the said enzyme;
the said composition being free of oxidation base.

2. Composition according to Claim 1, **characterized in that** the 2-electron oxidoreductase is chosen from pyranose oxidases, glucose oxidases, glycerol oxidases, lactate oxidases, pyruvate oxidases and uricases.

3. Composition according to either of Claims 1 and 2, **characterized in that** the 2-electron oxidoreductase is chosen from uricases of animal, microbiological or biotechnological origin.

4. Composition according to any one of the preceding claims, **characterized in that** the 2-electron oxidoreductase(s) represent(s) from 0.01 to 20% by weight relative to the total weight of the ready-to-use dye composition.

5. Composition according to Claim 4, **characterized in that** the 2-electron oxidoreductase(s) represent(s) from 0.1 to 5% by weight relative to the total weight of the ready-to-use dye composition.

6. Composition according to any one of Claims 3 to 5, **characterized in that** the donor (or substrate) for the said 2-electron oxidoreductase is chosen from uric acid and its salts.

7. Composition according to any one of the preceding claims, **characterized in that** the donor(s) represent(s) from 0.01 to 20% by weight relative to the total weight of the ready-to-use dye composition.

8. Composition according to Claim 7, **characterized in that** the donor(s) represent(s) from 0.1 to 5% by weight relative to the total weight of the ready-to-use dye composition.

9. Composition according to any one of the preceding claims, **characterized in that** the self-oxidizing dye(s) is (are) chosen from benzene, indole and indoline self-oxidizing dyes.

10. Composition according to Claim 9, **characterized in that** the benzene self-oxidizing dyes are chosen from the compounds of formula (I) below, and the addition salts thereof with an acid: 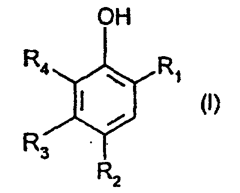 in which:
- R₁ represents a hydrogen atom, a C₁-C₄ alkyl radical or an amino radical,
- R₂ represents a C₁-C₄ alkyl, hydroxyl, amino, mono (C₁-C₄)alkylamino or di (C₁-C₄)alkylamino radical,
- R₃ represents a hydrogen atom or a hydroxyl or amino radical,
- R₄ represents a hydrogen atom or an amino radical;
it being understood that at least two of the radicals R₁
to R₄ represent, independently of each other, a hydroxyl, amino, mono(C₁-C₄)alkylamino or di(C₁-C₄)alkylamino radical.

11. Composition according to Claim 10, **characterized in that** the benzene self-oxidizing dyes of formula (I) are chosen from 1,2,4-trihydroxybenzene, 1-methyl-2,4,5-trihydroxybenzene, 2,4-diamino-6-methylphenol, 2-amino-4-methylaminophenol, 2,5-diamino-4-methylphenol, 2,6-diamino-4-diethylaminophenol and 2,6-diamino-1,4-dihydroxybenzene, and the addition salts thereof with an acid.

12. Composition according to Claim 9, **characterized in that** the indole and indoline self-oxidizing dyes are chosen from the compounds of formulae (II) and (III) below, and the addition salts thereof with an acid: 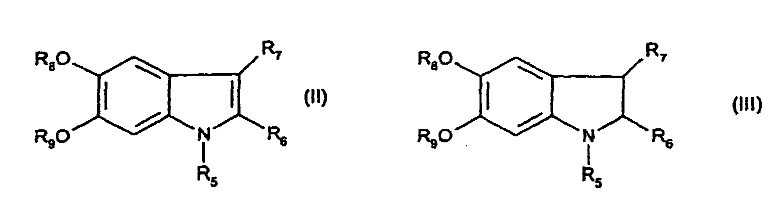 in which:
- R₅, R₇, R₈ and R₉, which may be identical or different, represent a hydrogen atom or a C₁-C₄ alkyl or C₁-C₄ acyl radical,
- R₆ represents a hydrogen atom, a C₁-C₄ alkyl radical or a carboxyl radical.

13. Composition according to Claim 12, **characterized in that** the self-oxidizing dyes of formula (II) are chosen from 5,6-dihydroxyindole, 2-methyl-5,6-dihydroxyindole, 3-methyl-5,6-dihydroxyindole, 1-methyl-5,6-dihydroxyindole, 2,3-dimethyl-5,6-dihydroxyindole, 5-methoxy-6-hydroxyindole, 5-acetoxy-6-hydroxyindole, 5,6-diacetoxyindole and 5,6-dihydroxyindole-2-carboxylic acid, and the addition salts thereof with an acid.

14. Composition according to Claim 12, **characterized in that** the self-oxidizing dyes of formula (III) are chosen from 5,6-dihydroxyindoline, 1-methyl-5,6-dihydroxyindoline and 1-ethyl-5,6-dihydroxyindoline, and the addition salts thereof with an acid.

15. Composition according to any one of the preceding claims, **characterized in that** the self-oxidizing dye(s) represent(s) from 0.0005 to 12% by weight relative to the total weight of the dye composition.

16. Composition according to Claim 15, **characterized in that** the self-oxidizing dye(s) represents) from 0.005 to 8% by weight relative to the total weight of the dye composition.

17. Composition according to any one of the preceding claims, **characterized in that** the addition salts with an acid are chosen from the hydrochlorides, hydrobromides, sulphates, tartrates, lactates and acetates.

18. Composition according to any one of the preceding claims, **characterized in that** the medium which is suitable for dyeing consists of water or of a mixture of water and at least one organic solvent.

19. Composition according to any one of the preceding claims, **characterized in that** it has a pH of between 5 and 11.

20. Composition according to any one of the preceding claims, **characterized in that** it contains at least one peroxidase.

21. Process for dyeing keratin fibres, and in particular human keratin fibres such as the hair, **characterized in that** at least one ready-to-use dye, composition as defined in any one of the preceding claims is applied to the said fibres for a period which is sufficient to develop the desired coloration.

22. Process according to Claim 21, **characterized in that** it includes a first step which consists in separately storing, on the one hand, a composition (A) comprising, in a medium which is suitable for dyeing, at least one self-oxidizing dye, and, on the other hand, a composition (B) containing, in a medium which is suitable for dyeing, at least one enzyme of 2-electron oxidoreductase type, in the presence of at least one donor for the said enzyme, and then in mixing them together at the time of use, after which this mixture is applied to the keratin fibres.

23. Multi-compartment dyeing device or "kit", **characterized in that** it comprises a first compartment containing composition (A) as defined in Claim 22 and a second compartment containing composition (B). as defined in Claim 22.

## Patentansprüche

1. Gebrauchsfertige Zusammensetzung zum oxidativen Färben von Keratinfasern und insbesondere menschlichen Keratinfasern, wie dem Haar, die in einem zum Färben geeigneten Träger enthält:
- mindestens einen selbstoxidierfähigen Farbstoff,
- mindestens ein Enzym vom Typ der Oxidoreduktasen (2 Elektronen), und
- mindestens einen Donor für das Enzym,
wobei die Zusammensetzung keine Oxidationsbase enthält.

2. Zusammensetzung nach Anspruch 1, **dadurch gekennzeichnet, dass** die Oxidoreduktase (2 Elektronen) unter den Pyranose-Oxidasen, Glucose-Oxidasen, Glycerin-Oxidasen, Lactat-Oxidasen, Pyruvat-Oxidasen und Uricasen ausgewählt ist.

3. Zusammensetzung nach einem der Ansprüche 1 oder 2, **dadurch gekennzeichnet, dass** die Oxidoreduktase (2 Elektronen) unter den Uricasen tierischer, mikrobiologischer oder biotechnologischer Herkunft ausgewählt ist.

4. Zusammensetzung nach einem der vorhergehenden Ansprüche, **dadurch gekennzeichnet, dass** die Oxidoreduktase(n) (2 Elektronen) 0,01 bis 20 Gew.-% des Gesamtgewichts der gebrauchsfertigen Farbmittelzusammensetzung ausmachen.

5. Zusammensetzung nach Anspruch 4, **dadurch gekennzeichnet, dass** die Oxidoreduktase(n) (2 Elektronen) 0,1 bis 5 Gew.-% des Gesamtgewichts der gebrauchsfertigen Farbmittelzusammensetzung ausmachen.

6. Zusammensetzung nach Anspruch 3 bis 5, **dadurch gekennzeichnet, dass** der Donor (oder das Substrat) für die Oxidoreduktase(n) (2 Elektronen) unter Harnsäure und ihren Salzen ausgewählt ist.

7. Zusammensetzung nach einem der vorhergehenden Ansprüche, **dadurch gekennzeichnet, dass** der Donor oder die Donoren 0,01 bis 20 Gew.-% des Gesamtgewichts der gebrauchsfertigen Farbmittelzusammensetzung ausmachen.

8. Zusammensetzung nach Anspruch 7, **dadurch gekennzeichnet, dass** der Donor oder die Donoren 0,1 bis 5 Gew.-% des Gesamtgewichts der gebrauchsfertigen Farbmittelzusammensetzung ausmachen.

9. Zusammensetzung einem der vorhergehenden Ansprüche, **dadurch gekennzeichnet, dass** der oder die selbstoxidierfähigen Farbstoffe unter den selbstoxidierfähigen Benzol-, Indol- und Indolin-Farbstoffen ausgewählt sind.

10. Zusammensetzung nach Anspruch 9, **dadurch gekennzeichnet, dass** die selbstoxidierfähigen Benzol-Farbstoffe unter den Verbindungen der folgenden Formel (I) und deren Additionssalzen mit einer Säure ausgewählt sind: 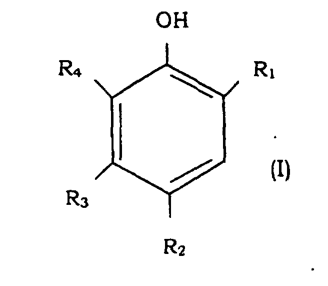 worin bedeuten:
- R₁ Wasserstoff, C₁₋₄-Alkyl oder Amino,
- R₂ C₁₋₄-Alkyl, Hydroxy, Amino, C₁₋₄-Monoalkylamino oder C₁₋₄-Dialkylamino,
- R₃ Wasserstoff, Hydroxy oder Amino, und
- R₄ Wasserstoff oder Amino,
- mit der Maßgabe, dass mindestens zwei der Gruppen R₁ bis R₄ unabhängig voneinander Hydroxy, Amino, C₁₋₄-Monoalkylamino oder C₁₋₄-Dialkylamino bedeuten.

11. Zusammensetzung nach Anspruch 10, **dadurch gekennzeichnet, dass** die selbstoxidierfähigen Benzol-Farbstoffe der Formel (I) unter 1,2,4-Trihydroxybenzol, 1-Methyl-2,4,5-trihydroxybenzol, 2,4-Diamino-6-methyl-phenol, 2-Amino-4-methylamino-phenol, 2,5-Diamino-4-methyl-phenol, 2,6-Diamino-4-diethylamino-phenol, 2,6-Diamino-1,4-dihydroxy-benzol und deren Additionssalze mit einer Säure ausgewählt sind.

12. Zusammensetzung nach Anspruch 9, **dadurch gekennzeichnet, dass** die selbstoxidierfähigen Indol- und Indolin-Farbstoffe unter den Verbindungen der folgenden Formeln (II) und (III) und deren Additionssalze mit einer Säure ausgewählt sind: 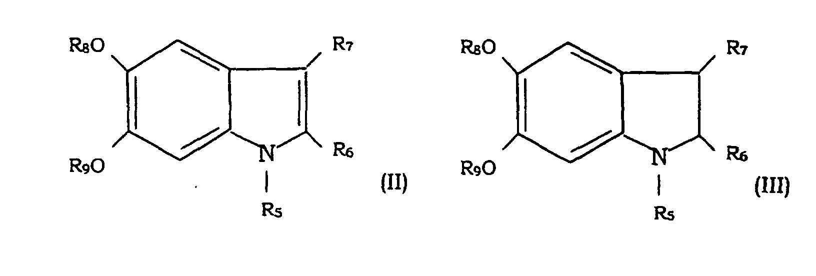 worin bedeuten:
- R₅, R_{7,} R₈ und R₉, die identisch oder voneinander verschieden sind, Wasserstoff, C₁₋₄-Alkyl oder C₁₋₄-Acyl, und
- R₆ Wasserstoff, C₁₋₄-Alkyl oder Carboxy.

13. Zusammensetzung nach Anspruch 12, **dadurch gekennzeichnet, dass** die selbstoxidierfähigen Farbstoffe der Formel (II) unter 5,6-Dihydroxy-indol, 2-Methyl-5,6-dihydroxy-indol, 3-Methyl-5,6-dihydroxy-indol, 1-Methyl-5,6-dihydroxy-indol, 2,3-Dimethyl-5,6-dihydroxy-indol, 5-Methoxy-6-hydroxy-indol, 5-Acetoxy-6-hydroxy-indol, 5,6-Diacetoxy-indol, 5,6-Dihydroxy-indol-2-carbonsäure und deren Additionssalzen mit einer Säure ausgewählt sind.

14. Zusammensetzung nach Anspruch 12, **dadurch gekennzeichnet**, die selbstoxidierfähigen Farbstoffe der Formel (III) unter 5,6-Dihydroxy-indolin, 1-Methyl-5,6-dihydroxy-indolin, 1-Ethyl-5,6-dihydroxy-indolin und deren Additionssalzen mit einer Säure ausgewählt sind.

15. Zusammensetzung nach einem der vorhergehenden Ansprüche, **dadurch gekennzeichnet, dass** der oder die selbstoxidierfähigen Farbstoffe 0,0005 bis 12 Gew.-% des Gesamtgewichts der Farbmittelzusammensetzung ausmachen.

16. Zusammensetzung nach Anspruch 15, **dadurch gekennzeichnet, dass** der oder die selbstoxidierfähigen Farbstoffe 0,005 bis 8 Gew.-% des Gesamtgewichts der Farbmittelzusammensetzung ausmachen.

17. Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

18. Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

19. Zusammensetzung nach einem der vorhergehenden Ansprüche, **dadurch gekennzeichnet, dass** sie einen pH-Wert im Bereich von 5 bis 11 aufweist.

20. Zusammensetzung nach einem der vorhergehenden Ansprüche, **dadurch gekennzeichnet, dass** sie mindestens eine Peroxidase enthält.

21. Verfahren zum Färben von Keratinfasern und insbesondere menschlichen Keratinfasern, wie dem Haar, **dadurch gekennzeichnet, dass** auf die Fasern während einer Zeitspanne, die zur Entwicklung der gewünschten Färbung ausreichend ist, mindestens eine gebrauchsfertige Farbmittelzusammensetzung nach einem der vorhergehenden Ansprüche aufgebracht wird.

22. Verfahren nach Anspruch 21, **dadurch gekennzeichnet, dass** es einen vorbereitenden Schritt umfasst, der darin besteht, einerseits eine Zusammensetzung (A), die in einem zum Färben geeigneten Medium mindestens einen selbstoxidierfähigen Farbstoff enthält, und andererseits eine Zusammensetzung (B) getrennt voneinander aufzubewahren, die in einem zum Färben geeigneten Medium mindestens ein Enzym vom Typ Oxidoreduktase (2 Elektronen) in Gegenwart mindestens eines Donors für das Enzym enthält, und diese bei der Anwendung zu vermischen, bevor das Gemisch auf die Fasern aufgebracht wird.

23. Vorrichtung mit mehreren Abteilungen oder "Kit" zum Färben, **dadurch gekennzeichnet, dass** sie eine erste Abteilung mit der in Anspruch 22 definierten Zusammensetzung (A) und eine zweite Abteilung mit der in Anspruch 22 definierten Zusammensetzung (B) enthält.
